# EUROPEAN PATENT SPECIFICATION

(11) **EP 2 822 541 B1**
(45) Date of publication and mention of the grant of the patent: **29.08.2018**
(21) Application number: 12766581.8
(22) Date of filing: 10.09.2012
(51) Int. Cl.: A61K 9/20, A61K 9/28, A61K 31/554

(54) **PHARMACEUTICAL COMPOSITION COMPRISING AN ATYPICAL ANTIPSYCHOTIC AGENT AND METHOD FOR THE PREPARATION THEREOF**
PHARMAZEUTISCHE ZUSAMMENSETZUNG MIT EINEM ATYPISCHEN ANTIPSYCHOTISCHEN WIRKSTOFF UND VERFAHREN ZUR HERSTELLUNG DAVON
COMPOSITION PHARMACEUTIQUE COMPRENANT UN AGENT ANTIPSYCHOTIQUE ATYPIQUE ET SON PROCÉDÉ DE PRÉPARATION

(43) Date of publication of application: 14.01.2015
(73) Proprietor: Pharmathen S.A., 15351 Pallini Attikis (GR)
(72) Inventor: KARAVAS, Evangelos, GR-153 51 Pallini Attikis (GR); KOUTRIS, Efthimios, GR-153 51 Pallini Attikis (GR); SAMARA, Vasiliki, GR-153 51 Pallini Attikis (GR); DIAKIDOU, Amalia, GR-153 51 Pallini Attikis (GR); PAPANIKOLAOU, Georgia, GR-153 51 Pallini Attikis (GR); BARMPALEXIS, Panagiotis, GR-153 51 Pallini Attikis (GR)
(86) International application number: PCT/EP2012/003781
(87) International publication number: WO 2014/037022

(56) References cited:
- EP-A2- 2 153 834
- WO-A1-2010/066342
- US-A1- 2011 052 648
- US-A1- 2011 151 002

## Description

### TECHNICAL FIELD OF THE INVENTION

The present invention relates to a stable pharmaceutical formulation for oral administration containing a therapeutically effective quantity of an atypical antipsychotic agent such as Quetiapine or pharmaceutical acceptable salt and a method for the preparation thereof.

### BACKGROUND OF THE INVENTION

Atypical antipsychotics (also known as second generation antipsychotics) are a class of prescription medications used for the treatment of psychiatric conditions. Said drugs have found favor among clinicians and are now considered to be first line treatment for schizophrenia and are gradually replacing the typical antipsychotic agents. Both generations of medication tend to block receptors in the brain's dopamine pathways, but atypical differ from typical antipsychotics in that they are less likely to cause extrapyramidal motor control disabilities in patients, which include unsteady Parkinson's disease-type movements, body rigidity and involuntary tremors. These abnormal body movements can become permanent even after medication is stopped.

Quetiapine constitutes one of the most commonly used atypical antipsychotic agents. It is used to treat symptoms of schizophrenia, manic episodes associated with bipolar disorder, and depressive episodes associated with bipolar disorder. Quetiapine is also used together with antidepressant medications to treat major depressive disorder in adults. It works by affecting the actions of certain chemicals in the brain known as neurotransmitters. These brain chemicals are believed to be out of balance in individuals with schizophrenia or bipolar disorder, and Quetiapine helps get them back into balance. As a result, symptoms are reduced or alleviated altogether.

Quetiapine is a compound of particular interest since it may be used as an antipsychotic agent with a substantial reduction in the potential to cause side effects such as acute dystonia, acute dyskinesia, pseudo-Parkinsonism and tardive dyskinesia which side-effects may result from the use of another anti-psychotics or neuroleptics.

Quetiapine is a psychotropic agent that belongs to the class of drugs known as dibenzothiazepines. The chemical name of Quetiapine is 11-[4-[2-(2-hydroxyethoxy)ethyl]-1-piperazinyl]dibenzo[b,f][1,4]thiazepine. The molecular formula is C₄₂H₅₀N₆O₄S₂•C₄H₄O₄ corresponding to a molecular weight of 883.11 (for its fumarate salt). It is a white to off-white crystalline powder which is moderately soluble in water.

EP-B-0907364 discloses sustained release formulations of Quetiapine comprising a gelling agent and one or more pharmaceutical acceptable excipients.

US-A-2005/158383 discloses controlled release formulations comprising a matrix containing Quetiapine or pharmaceutical acceptable salt and a wax material such as carnauba wax, glyceryl behenate.

WO-A-2007/133583 discloses a zero order modified release dosage form comprising a matrix core containing a hydrophobic material and a water soluble pharmaceutical agent and a modified release coating surrounding the matrix core.

WO2010/066342 A1 discloses extended release quetiapine formulations comprising the drug and non-swellable anionic polymer particles, such as Acryl-Eze (comprising polymethacrylate ethylacrylate polymer with a ratio of free carboxyl groups to the ester groups 1:1). The pharmaceutical composition can be in the form of tablets, manufactured by wet or dry granulation. The tablets may be coated. Although each of the patents above represents an attempt to overcome the problems associated with pharmaceutical compositions comprising water soluble active ingredients, there still exists a need for a stable pharmaceutical composition which avoids such problems.

### SUMMARY OF THE INVENTION

It is, therefore, an object of the present invention to provide a stable oral solid dosage formulation for oral administration containing a dibenzothiazepine agent, and in particular Quetiapine or pharmaceutical acceptable salt thereof, as an active ingredient, which overcomes the deficiencies of the prior art and provides a uniform and constant rate of release over an extended period of time.

It is another object of the present invention to provide an extended release pharmaceutical composition for oral administration comprising Quetiapine or pharmaceutical acceptable salt thereof, as an active ingredient, which is bioavailable, with sufficient self-life and good pharmacotechnical properties.

Another aspect of the present invention is to provide an oral solid dosage formulation comprising Quetiapine or pharmaceutical acceptable salt thereof, which overcomes the problems related to the use of gelling agents such as dose dumping and food effects on the drug bioavailability.

A major object of the present invention is the selection of an ionic non-gelling matrix polymer which incorporates the active ingredient in order to achieve the appropriate dissolution profile and stability for the finished dosage form. Said dosage form affords predictable and reproducible drug release rates in order to achieve better treatment to a patient.

Further object of the present invention is to provide a solid dosage form for oral administration containing Quetiapine or pharmaceutical acceptable salt thereof, which avoids anv disadvantage of formulations comprising water soluble active ingredients.

A further approach of the present invention is to provide an extended release dosage form containing Quetiapine or pharmaceutical acceptable salt thereof which is manufactured through a fast, simple and cost-effective process.

In accordance with the above objects of the present invention, an extended release pharmaceutical composition for oral administration is provided comprising Quetiapine or a pharmaceutical acceptable salt thereof, as an active ingredient and an effective amount of the same matrix forming non-gelling, non-swellable, methacrylic acid-based enteric polymer both in the composition core and coating, wherein said enteric polymer is an anionic copolymer based on methacrylic acid and ethyl acrylate wherein the ratio of its free carboxyl groups to ester groups is 1:1. According to another embodiment of the present invention, a process for the preparation of an extended release solid dosage form is provided wherein said process comprises the steps:
- weighing and sieving Quetiapine or a pharmaceutical acceptable salt thereof and all the pharmaceutically acceptable excipients of the composition;
- blending Quetiapine or a pharmaceutical acceptable salt thereof, with an effective amount of a matrix forming non-gelling, non-swellable methacrylic acid-based enteric polymer and at least one water soluble, non gelling sugar, if present, until complete homogeneity;
- kneading the above mixture with water and then drying the wetted mass;-sieving the dried mass, adding to the sieved mixture at least one pharmaceutically acceptable excipient selected from glidants and/or lubricants and mixing until uniformity;
- compressing the resulted mixture into a tablet dosage form;
- applying a film of the same enteric polymer as in the core and, optionally, at least one further excipient selected from plasticizers, colorants, dyes, pigments, surfactants, or combinations thereof on the tablet dosage form.

Other objects and advantages of the present invention will become apparent to those skilled in the art in view of the following detailed description.

### BRIEF DESCRIPTION OF THE DRAWINGS

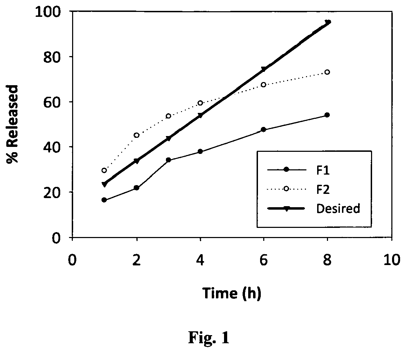

Fig 1 shows the dissolution profiles of Formulations F1 and F2 compared with the desired pH independent profile.

### DETAILED DESCRIPTION OF THE INVENTION

For the purposes of the present invention, a pharmaceutical composition comprising an active ingredient (e.g. Quetiapine or pharmaceutical acceptable salt thereof) is considered to be "stable" if said ingredient degrades less or more slowly than it does on its own and/or in known pharmaceutical compositions.

As already mentioned the main object of the present invention is to provide an extended release composition of Quetiapine or pharmaceutical acceptable salt thereof that is simple to manufacture, bioavailable, cost effective, stable and possesses good pharmacotechnical properties and linearitv.

An obstacle related to the development of a pharmaceutical composition according to the above mentioned objects, is the phenomenon known as dose dumping. In fact, dose dumping is common in extended release formulations of water soluble drugs, such as Quetiapine. More specifically such phenomenon is particularly intensified when gelling agents are comprised in extended release compositions of water soluble drugs. Dose dumping is defined as: "Unintended, rapid drug release in a short period of time of the entire amount or a significant fraction of the drug contained in a modified release dosage form." An extended release dosage form is intended to release the drug in desired concentrations for a prolonged period of time. A dosage form is said to be dose dumped when there is an excess release of drug at a particular time interval other than the stated or required amount. This results in higher systemic drug concentrations that may produce adverse effects or even drug-induced toxicity.

Another problem related to gelling matrix systems is the pronounced effect of food on drug bioavailability. The term "bioavailability" is used to describe the fraction of drug dose which reaches the systemic circulation unchanged. Many of the factors which influence bioavailability can be changed by food, both "acutely", if a drug is taken with a meal, and "chronically", where regularly consumed food items influence drug disposition. The nature of these interactions is complicated, and is influenced by the quantity and composition of food. It should also be noted that as well as changing the pharmacokinetics of drugs, food can alter, as well, their pharmacodynamic effects.

It has been found that the object of the present invention is achieved when non-gelling and non-swellable excipients are incorporated into the formulation. Many weakly basic drugs, such as Quetiapine or pharmaceutical acceptable salt thereof, demonstrate pH-dependent solubility in the gastrointestinal tract, leading to variable dissolution rates. The rate at which a drug is dissolved is proportional to the solubility of the drug in the medium, and hence, different drug release rates could result in variable oral absorption and bioavailability problems. Therefore, preparation of a pH-independent constant extended release dosage form is desirable for a reliable drug therapy.

Several attempts have been made to overcome pH-dependent variability in sustained release formulations of weakly basic drugs. The present invention ensures pharmaceutical availability of Quetiapine or pharmaceutical acceptable salt thereof from extended release dosage forms using micro-environmental pH modulators and/or materials with pH-dependent solubility, such as polymethacrylates, in order to create a system with pH-independent dissolution profile. These formulations provide constant release of Quetiapine during the transit through the gastro-intestinal tract in spite of the variation in pH values.

Enteric polymers or co-polymers, such as polymethacrylates, can contribute to the retardation of the release phenomenon in the stomach (by adjusting tablet's micro-environmental pH) while acting as pore-forming agents at higher pH values (intestine). Moreover, the same type of enteric polymers or copolymers are used as coating agents to enhance pH-independent dissolution behaviour when the formulation is transferred from stomach to small intestine.

Ionic non-gelling matrix polymers are ionic polymers that do not swell to form a gel when exposed to an aqueous medium. Typically, they have pH dependent solubility. That means that are practically insoluble at one fluid pH but soluble at another. As the composition travels along the GI tract, the pH increases and thereby allows for the matrix to dissolve and continue releasing the active ingredient in a controlled manner. As the composition travels further in the GI tract where the pH further increases, the ionic non-gelling matrix polymer will be solubilised thereby releasing the remaining active agent. Such a mechanism allows for the efficient and more complete release of the active agent and limits the amount of active agent trapped in the composition. Furthermore, the complete dissolution of the ionic non-gelling matrix polymer is delaved until after it is exposed to the pH at which it is soluble. The ionic non-gelling matrix polymer can be present in an amount from about 5 to about 85wt%, specifically from about 10 to about 50wt %, more specifically from about 10 to 20wt % of the total weight of the controlled release composition.

The ionic non-swellable polymer can be used as a pure polymer or as blended ready to use product such as Sureteric, Acryl-Eze or the like. Acryl-Eze is a preblended product containing polymethacrylate-ethylacrylate polymer (Eudragit L100-55), plasticizer and pigments.

According to the present invention, the ionic non-swellable polymer is a methacrylic acid-based enteric polymer. Suitable methacrylic acid-based enteric polymers are dissolved at pH value of 5.5 or above. Suitable specific examples of the methacrylic acid-based enteric polymer include methacrylic acid copolymer LD, methacrylic acid copolymer L, methacrylic acid copolymer S, and the like. As methacrylic acid-based enteric polymers, commercial available products such as Eudragit L100-55 may be used as a dry methacrylic acid copolymer LD, Eudragit L100 as a methacrylic acid copolymer L, and Eudragit S100 as a methacrylic acid copolymer S. These enteric polymers may be used singly or in a combination of two or more. The methacrylic acid-based enteric polymer used for the pharmaceutical solid preparation of the invention is preferably dissolved at a pH of 5.5 or above.

The extended release matrix of the present invention may further comprise additional pharmaceutically acceptable excipients which are soluble non-gelling excipient and can act as pore formers allowing the formation of channels in the matrix thereby increasing the rate of active ingredient diffusion from the matrix at the first acidic pH. Exemplary soluble non-gelling pharmaceutically acceptable excipients include, but are not limited to, lactose, sucrose, dextrose, glucose, maltose, sorbitol, and combinations comprising at least one of the foregoing.

The soluble non-gelling pharmaceutically acceptable excipient can be present in the matrix in an amount from about 1% to 70 wt%, specifically from 10 to about 60wt% and more specifically 20 to 50 wt% based on the total weight of the controlled release composition.

Moreover, the pharmaceutical compositions of the present invention may also contain one or more additional formulation excipients such as diluents, disintegrants, binders, lubricants, glidants and flavouring agents, provided that they are compatible with the active ingredient of the composition in order to increase the stability of the drug and the self-life of the pharmaceutical product.

The pharmaceutical composition can be coated with a functional film coating giving the dosage forms the required physical or biopharmaceutical properties, decreased permeability to moisture and other gases such as oxygen, taste or colour masking and/or smoothening of the surface for easier swallowing in case of tablets. By "functional coating", it is meant to include a coating that modifies the release properties of the total formulation, for example a sustained release coating. The coating material includes the same matrix forming non-gelling, non-swellable, methacrylic acid-based enteric polymer as the composition core, wherein said enteric polymer is an anionic copolymer based on methacrylic acid and ethyl acrylate wherein the ratio of its free carboxyl groups to ester groups is 1:1. The coating material may comprise other components, such as for example, pharmaceutically acceptable nlasticizers, colorants, dyes, pigments, surfactants, or combinations thereof.

The extended release formulations of the present invention are prepared by wet granulation in order to improve flow and compressibility of powders and to prevent segregation of the blend components. Said method is used to convert a powder mixture into granules having suitable flow and cohesive properties for tabletting.

The manufacturing process according to the present invention comprises the following steps:
- The excipients that are present in both internal and external phases as well as the active substance are weighed and sieved.
- Ingredients of the internal phase, namely a methacrylic acid-based enteric polymer and at least one more excipient selected from the group consisting of diluents, lubricants, glidants, binders or fillers and the active substance are mixed in a blender. Blending is performed until uniformity of the powder;
- The granulation liquid, water, is added to the mixture obtained from the previous step;
- The wetted mass is dried;
- The granules are sifted;
- The mixture resulted from the previous step is mixed with a glidant and a lubricant;
- The powder mixture is compressed into the desirable tablet dosage form;
- The tablets are coated with an enteric polymer comprising at least one more excipient selected from the group consisting of plasticizers, colorants, dyes, pigments, surfactants, or combinations thereof.

The composition according to the present invention is capable of extending the release of the active substance from at least 12 hours, preferably to at least 16 hours and most preferably to at least 24 hours.

A number of sustained release compositions comprising different excipients were tested as presented in the following examples to achieve the optimal properties with respect to the objects of the present invention.

### EXAMPLES

### Example 1 (not falling under the scope of the claims)

**Table 1: Qualitive & quantitive composition of example 1 (F1)**

| **Ingredients** | **% content** |
|---|---|
| Quetiapine Fumarate | 55.69 |
| Eudragit L100 | 24.21 |
| Lactose | 12.11 |
| Tartaric acid | 0.73 |
| Talc | 7.26 |
| **Total Weight uncoated** | **100** |

All the excipients and the active substance of composition of example 1 (F1) where weighted and sifted. Quetiapine fumarate, Eudragit L100, Lactose and Tartaric acid where added in a blender and mixed until uniformity. Subsequently, the granule was wet granulated with water, mixed and dried. The dried granule was sifted and lubricated with talc. Finally, the granule was compressed into the desirable tablet form and coated with Eudragit L12.5. The flow properties of the powder mix where satisfactory but the compressibility was poor. The hardness of tablets (measured as resistance to crushing) was 65 ±5.7 N while the percentage of friability was 1.2 ± 0.3 %.

### Example 2 (not falling under the scope of the claims)

**Table 2: Qualitive & quantitive composition of example 2 (F2)**

| **Ingredients** | **% content** |
|---|---|
| Quetiapine Fumarate | 64.61 |
| Eudragit L100 | 28.09 |
| Tartaric acid | 0.84 |
| Talc | 6.46 |
| **Total Weight uncoated** | **100** |

All the excipients and the active substance of composition of example 2 (F2) where weighted and sifted. Quetiapine fumarate, Eudragit L100 and Tartaric acid where added in a blender and mixed until uniformity. Subsequently, the granule was wet granulated with water, mixed and dried. The dried granule was sifted and lubricated with talc. Finally, the granule was compressed into the desirable tablet form and coated with Eudragit L12.5. Compared to composition of example 1 (F1), tablets of example 2 (F2) did not contain Lactose, while the percentage content of Talc in the core was lower. The pharmacotechnical characteristics of produced tablets remained unsatisfactory.

The dissolution tests conducted on compositions F1 and F2 did not give satisfactory results as well (Fig. 1). Comparison of the two in vitro dissolution profiles for compositions F1 and F2 showed significant differences in the amount of Quetiapine released in 1 and 8 h, respectively. Since the conditions and the weight gain of tablets during coating remained constant, differences between the two dissolution profiles were attributed to the type and amount of excipients used in the core. Analytically, the increased amounts of Quetiapine released in acidic medium for composition F2 were attributed to the reduction of talc in the core, which resulted to a more hydrophilic matrix leading to increased drug release. Furthermore, the slow dissolution rate of Quetiapine after two hours for both compositions F1 and F2 indicated that the addition of tartaric acid as a tablet micro-environmental pH modifier was not appropriate to achieve the desired dissolution rates.

Furthermore, a compatibility study conducted between Quetiapine and tartaric acid blends at accelerated storage conditions indicated significant compatibility problems.

### Example 3

Eudragit L100-55 was selected instead of Eudragit L100 for both tablet core and coating layer. Compared to Eudragit L100, Eudragit L100-55, which is also an anionic copolymer, dissolves at a slightly lower pH value (about 5.5) and indicates a significantly higher dissolution rate in neutral and alkaline mediums. Additionally, two sugar types was decided to be added in the core as water soluble non-gelling excipients in order to facilitate release of the active substance from the core at the initial acidic pH of the stomach. The first sugar type was decided to be lactose and the other one selected from maltose or dextrose.

In order to screen from a variety of formulation factors an experimental 2⁴ full factorial design with two centre points was initiated. The factors involved were the type and the amount of two sugars (dextrose and maltose), the amount of Eudragit L100-55 and the amount of lactose. Two dissolution parameters, namely the percentage dissolved drug at 1 hour and 8 hours (Y1h and Y8h), were selected as responses.

All tested tablets showed extended release profiles of Quetiapine. The profiles indicated a combination of two dissolution mechanisms depending on the pH of the dissolution medium. In the acidic medium, where Quetiapine indicates higher solubility as a weakly basic drug, tablets acted as reservoir type matrices (diffusion through the coating layer). The release of the drug through the enteric coating depended on the hydrophilicity of the core and the thickness of the coating layer. Since the conditions and the weight gain of tablets during coating remained constant, differences between the dissolution profiles of the experiments were attributed to differences in the core. On the contrary, at higher pH values, where Quetiapine indicates lower solubility and coating is freely soluble, erosion was the main release mechanism. The erosion rate of the matrix was mainly controlled by the solubility of the Quetiapine and the hydrophilicity of the core excipients.

ANOVA analysis indicated that three out of four examined factors, namely the amount of sugar, Eudragit L100 55 and lactose had statistically significant influence on the percentage release of Quetiapine in 1 hour while the type and the amount of sugar, as well as the amount of Eudragit L100 55 had significant influence on the percentage release of Quetiapine in 8 hours.

Linear-regression models analysis indicated that the amount of sugar and the amount of Eudragit L100-55 exhibited an antagonistic effect for both Y₁ₕ and Y₈ₕ; while increasing amounts of lactose led to decreasing Y₁ₕ values. The experimental analysis showed that the amount of drug released in the first hour can be controlled through the selected formulation factors and coating layer.

The preferred composition according to the present invention (F3) is illustrated in Table 3 below:

**Table 3: Qualitive & quantitive composition of example 3 (F3)**

| **Ingredients** | **% content** |
|---|---|
| Quetiapine Fumarate | 43.81 |
| Eudragit L100 55 | 12.38 |
| Maltose | 28.58 |
| Lactose | 9.52 |
| Talc | 3.81 |
| Magnesium stearate | 1.90 |
| **Total Weight uncoated** | **100** |

Composition of example 3 (F3) was prepared according to the following manufacturing process:
- The active substance and all excipients of internal and external phase were weighted and sieved;
- Quetiapine fumarate and the excipients of the internal phase, Eudragit L 100-55, Lactose and Maltose, were added in a blender and mixed until complete homogeneity;
- Kneading of the above mixture with water and then drying the wetted mass;
- Sieving the dried mass and adding to the sieved mixture the external phase excipients, talc and magnesium stearate and mixing until uniformity;
- Compressing the resulted mixture into a tablet dosage form;
- Applying a film-coating of Eudragit L100-55 and Citrofol on the core.

The in-vitro drug release experiments of the tablets above showed extended release profiles. The percentage release of Quetiapine in 1 and 8 hours was approximately 22% and 84.9%. Fitting of the zero order release kinetic model on the mean dissolution profiles of the tested formulation showed good correlation (R² = 0.968). Also the similarity factor value was 62.77 that indicated good similarity of the formulations with the desirable ph-independent profile

The high correlation coefficient value for zero order release model (R²), the high value of similarity factor (f₂), along with the improved pharmacotechnical characteristics and stability of composition F3, indicates is able to provide a constant, pH-independent extended release of Quetiapine.

## Claims

1. An extended release pharmaceutical composition for oral administration comprising Quetiapine or a pharmaceutical acceptable salt thereof, as an active ingredient and an effective amount of the same matrix forming non-gelling, non-swellable, methacrylic acid-based enteric polymer both in the composition core and coating, wherein said enteric polymer is an anionic copolymer based on methacrylic acid and ethyl acrylate wherein the ratio of its free carboxyl groups to ester groups is 1:1.

2. The pharmaceutical composition according to claim 1, comprising at least one water soluble non gelling sugar in the matrix.

3. The pharmaceutical composition according to claim 2, wherein the at least one water soluble non gelling sugar is selected from lactose, sucrose, dextrose, glucose, maltose, sorbitol or combinations thereof

4. The pharmaceutical composition according to claim 1, wherein the non-gelling, non-swellable matrix forming polymer is present in an amount from 5 to 85wt%, of the total weight of the composition.

5. The pharmaceutical composition according to claim 4, wherein the non-gelling, non-swellable matrix forming polymer is present in an amount from10 to 50wt % and more specifically from 10 to 20wt % of the total weight of the composition.

6. The pharmaceutical composition according to claim 4, wherein the non-gelling, non-swellable matrix forming polymer is present in an amount from 10 to 20wt % of the total weight of the composition.

7. The pharmaceutical composition according to claim 2 or 3, wherein the at least one water soluble non gelling sugar is present in an amount from about 1% to 70 wt%based on the total weight of the composition

8. The pharmaceutical composition according to claim 7, wherein the at least one water soluble non gelling sugar is present in an amount from 10 to 60 wt% based on the total weight of the composition.

9. The pharmaceutical composition according to claim 7, wherein the at least one water soluble non gelling sugar is present in an amount from 20 to 50 wt% based on the total weight of the composition

10. The pharmaceutical composition according to any preceding claim, wherein it further comprises other pharmaceutically acceptable excipients such as glidants and/or lubricants.

11. The pharmaceutical composition according to any preceding claim, wherein the coating comprises at least one further excipient selected from plasticizers, colorants, dyes, pigments, surfactants, or combinations thereof.

12. A process for the preparation of an extended release solid dosage form as defined in any claim from 1 to 11 wherein said process comprises the steps:
- weighing and sieving Quetiapine or a pharmaceutical acceptable salt thereof and all the pharmaceutically acceptable excipients of the composition;
- blending Quetiapine or a pharmaceutical acceptable salt thereof, with an effective amount of a matrix forming non-gelling, non-swellable methacrylic acid-based enteric polymer and at least one water soluble, non gelling sugar, if present, until complete homogeneity;
- kneading the above mixture with water and then drying the wetted mass;
- sieving the dried mass, adding to the sieved mixture at least one pharmaceutically acceptable excipient selected from glidants and/or lubricants and mixing until uniformity;
- compressing the resulted mixture into a tablet dosage form;
- applying a film of the same enteric polymer as in the core and, optionally, at least one further excipient selected from plasticizers, colorants, dyes, pigments, surfactants, or combinations thereof on the tablet dosage form.

## Patentansprüche

1. Pharmazeutische Zusammensetzung mit verlängerter Freisetzung zur oralen Verabreichung, die Quetiapin oder ein pharmazeutisch verträgliches Salz davon, als aktiven Bestandteil und eine wirksame Menge des gleichen matrixbildenden nicht-gelierenden, nicht-quellbaren, auf Methacrylsäure basierenden enterischen Polymers, sowohl in der Zusammensetzung als auch in der Zusammensetzung Beschichtung umfassend, wobei das enterische Polymer ein anionisches Copolymer auf Basis von Methacrylsäure und Ethylacrylat ist, wobei das Verhältnis seiner freien Carboxylgruppen 1: 1 beträgt.

2. Pharmazeutische Zusammensetzung nach Anspruch 1, die mindestens einen wasserlöslichen nicht-gelierenden Zucker in der Matrix umfassend.

3. Pharmazeutische Zusammensetzung nach Anspruch 2, wobei der mindestens eine wasserlösliche, nicht gelierende Zucker aus Lactose, Saccharose, Dextrose, Glucose, Maltose, Sorbit oder Kombinationen davon ausgewählt ist.

4. Pharmazeutische Zusammensetzung nach Anspruch 1, wobei das nicht gelierende, nicht quellbare Matrix bildende Polymer in einer Menge von 5 bis 85 Gew .-% des Gesamtgewichts der Zusammensetzung vorliegt.

5. Pharmazeutische Zusammensetzung nach Anspruch 4, wobei das nichtgelierende, nicht quellbare, Matrix bildende Polymer in einer Menge von 10 bis 50 Gew .-% und insbesondere von 10 bis 20 Gew .-% des Gesamtgewichts der Zusammensetzung vorliegt.

6. Pharmazeutische Zusammensetzung nach Anspruch 4, wobei das nicht gelierende, nicht quellbare, Matrix bildende Polymer in einer Menge von 10 bis 20 Gew .-% des Gesamtgewichts der Zusammensetzung vorliegt.

7. Pharmazeutische Zusammensetzung nach Anspruch 2 oder 3, wobei der mindestens eine wasserlösliche, nicht gelierende Zucker in einer Menge von etwa 1 bis 70 Gew .-% des Gesamtgewichts der Zusammensetzung vorliegt.

8. Pharmazeutische Zusammensetzung nach Anspruch 7, wobei der mindestens eine wasserlösliche, nicht gelierende Zucker in einer Menge von etwa 10 bis 60 Gew .-% des Gesamtgewichts der Zusammensetzung vorliegt.

9. Pharmazeutische Zusammensetzung nach Anspruch 7, wobei der mindestens eine wasserlösliche, nicht gelierende Zucker in einer Menge von etwa 20 bis 50 Gew .-% des Gesamtgewichts der Zusammensetzung vorliegt.

10. Pharmazeutische Zusammensetzung nach einem der vorhergehenden Ansprüche, die ferner andere pharmazeutisch annehmbare Hilfsstoffe, wie Gleitmittel und / oder Schmiermittel, umfaßt.

11. Pharmazeutische Zusammensetzung nach einem der vorhergehenden Ansprüche, wobei die Beschichtung mindestens einen weiteren Hilfsstoff umfasst, der aus Weichmachern, Farbstoffen, Farbe, Pigmenten, Tensiden oder Kombinationen davon ausgewählt ist.

12. Verfahren zur Herstellung einer festen Darreichungsform mit verlängerter Freisetzung nach einem der Ansprüche 1 bis 11, wobei das Verfahren die folgenden Schritte umfaßt:
- Quetiapin oder ein pharmazeutisch verträgliches Salz davon und alle pharmazeutisch annehmbaren Hilfsstoffe in der Zusammensetzung wiegen und sieben.
- Quetiapin oder eines pharmazeutisch verträglichen Salz davon, mit einer wirksamen Menge einer Matrix, die nicht-gelierendes, nicht-geschwefeltes enterisches Polymer auf Methacrylsäurebasis bildet, und mindestens einem wasserlöslichen, nicht gelierenden Zucker, falls vorhanden, bis zur vollständigen Homogenisierung mischen;
- der obigen Mischung mit Wasser kneten und dann der angefeuchteten Masse trocknen;
- der getrockneten Masse sieben; von mindestens einem pharmazeutisch annehmbaren Hilfsstoffe, ausgewählt aus Gleitmitteln und / oder Schmiermittel, zu der gesiebten Mischung zugaben und bis zur Gleichförmigkeit mischen;
- der resultierenden Mischung zu einer Tabletten-Dosierungsform komprimieren;
- einer Folie aus dem gleichen magensaftresistenten Polymer wie im Kern aufbringen und gegebenenfalls mindestens einem weiteren Hilfsstoff, ausgewählt aus Weichmachern, Farbstoffen, Farbe, Pigmenten, Tensiden oder deren Kombinationen auf der Tablettenform aubringen.

## Revendications

1. Une composition pharmaceutique à libération prolongée pour administration orale comprenant la Quetiapine ou un sel pharmaceutiquement acceptable de celle-ci, en tant que principe actif, et une quantité efficace de polymère entérique à base d'acide méthacrylique non-gélifiant, non-gonflant formant la même matrice tant au niveau du noyau de la composition que du revêtement, dans laquelle le dit polymère entérique est un copolymère anionique à base d'acide méthacrylique et d'acrylate d'éthyle où le rapport entre ses groupes carboxyliques libres et ses groupes esters est de 1 : 1.

2. La composition pharmaceutique selon la revendication 1, comprenant au moins un sucre soluble dans l'eau et non-gélifiant dans la matrice.

3. La composition pharmaceutique selon la revendication 2, dans laquelle ledit au moins un sucre soluble dans l'eau et non-gélifiant est sélectionné parmi le lactose, le sucrose, le dextrose, le glucose, le maltose, le sorbitol ou combinaisons de ceux-ci.

4. La composition pharmaceutique selon la revendication 1, dans laquelle le polymère non-gélifiant, non-gonflant formant matrice est présent en une quantité de 5 à 85% en poids du poids total de la composition.

5. La composition pharmaceutique selon la revendication 4, dans laquelle le polymère non-gélifiant, non-gonflant formant matrice est présent en une quantité de 10 à 50% en poids et plus spécifiquement de 10 à 20% en poids du poids total de la composition.

6. La composition pharmaceutique selon la revendication 4, dans laquelle le polymère non-gélifiant, non-gonflant formant matrice est présent en une quantité de 10 à 20% en poids du poids total de la composition.

7. La composition selon la revendication 2 ou 3, dans laquelle ledit au moins un sucre soluble dans l'eau et non-gélifiant est présent en quantité de 1 à 70% en poids basé sur le poids total de la composition.

8. La composition pharmaceutique selon la revendication 7, dans laquelle ledit au moins un sucre soluble dans l'eau et non-gélifiant est présent en quantité de 10 à 60% en poids basé sur le poids total de la composition.

9. La composition pharmaceutique selon la revendication 7, dans laquelle ledit au moins un sucre soluble dans l'eau et non-gélifiant est présent en quantité de 20 à 50% en poids basé sur le poids total de la composition.

10. La composition pharmaceutique selon l'une quelconque des revendications précédentes, dans laquelle sont compris en outre d'autres excipients pharmaceutiquement acceptables comme des agents de glissement et/ou des lubrifiants.

11. La composition pharmaceutique selon l'une quelconque des revendications précédentes, dans laquelle le revêtement comprend au moins un excipient supplémentaire sélectionné parmi des plastifiants, colorants, teintures, pigments, tensioactifs, ou combinaisons de ceux-ci.

12. Un procédé pour la préparation d'une forme posologique solide à libération prolongée telle que définie dans l'une quelconque des revendications de 1 à 11, dans lequel ledit procédé comprend les étapes suivantes :
- peser et tamiser la Quetiapine ou un sel pharmaceutiquement acceptable de celle-ci et tous les excipients pharmaceutiquement acceptables de la composition;
- mélanger la Quetiapine ou un sel pharmaceutiquement acceptable de celle-ci avec une quantité efficace de polymère entérique formant la matrice non-gélifiant, non-gonflant à base d'acide méthacrylique et au moins un sucre soluble dans l'eau, non-gélifiant, si celui-ci est présent, jusqu'à homogénéité complète;
- malaxer le mélange ci-dessus avec de l'eau et puis sécher la masse mouillée;
- tamiser la masse séchée, ajouter au mélange tamisé au moins un excipient pharmaceutiquement acceptable sélectionné parmi des glissants et/ou lubrifiants et mélanger jusqu'à uniformité;
- compresser le mélange résultant sous la forme d'un comprimé;
- appliquer un film du même polymère entérique comme au niveau du noyau et, éventuellement, au moins un excipient supplémentaire sélectionné parmi des plastifiants, colorants, teintures, pigments, tensioactifs ou combinaisons de ceux-ci sur le comprimé.
